# EUROPÄISCHE PATENTSCHRIFT

(11) **EP 2 573 077 B1**
(45) Veröffentlichungstag und Bekanntmachung des Hinweises auf die Patenterteilung: **04.10.2017**
(21) Anmeldenummer: 12197983.5
(22) Anmeldetag: 21.07.2006
(51) Int. Cl.: C07D 275/03

(54) **VERFAHREN ZUR HERSTELLUNG VON 3,4-DICHLOR-N-(2-CYANO-PHENYL)- 5-ISOTHIAZOLCARBOXAMID**
METHOD FOR PRODUCING 3,4-DICHLOR-N-(2-CYANO-PHENYL)-5-ISOTHIAZOLE CARBOXAMIDE
PROCÉDÉ DE FABRICATION DE 3,4-DICHLORO-N-(2-CYANO-PHÉNYL)- 5-ISOTHIAZOLCARBOXAMIDE

(30) Priorität: 29.07.2005 DE 102005035617
(43) Veröffentlichungstag der Anmeldung: 27.03.2013
(62) Teilanmeldung aus: 06828798.6
(73) Patentinhaber: Bayer CropScience AG, 40789 Monheim am Rhein (DE)
(72) Erfinder: Himmler, Thomas, 51519 Odental (DE)
(74) Vertreter: Colling-Hendelkens, Miriam

(56) Entgegenhaltungen:
- WO-A-2004/002968

## Beschreibung

Die vorliegende Erfindung betrifft ein neues Verfahren zur Herstellung und Isolierung von 3,4-Dichlor-N-(2-cyano-phenyl)-5-isothiazolcarboxamid, das als Wirkstoff mit mikrobiziden Eigenschaften verwendbar ist.

Es ist bekannt, dass man 3,4-Dichlor-N-(2-cyano-phenyl)-5-isothiazolcarboxamid der allgemeinen Formel (I) 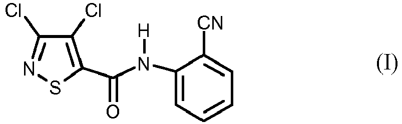 erhält, wenn man 3,4-Dichlorisothiazol-5-carbonsäurechlorid mit 2-Cyano-anilin umsetzt (vgl. WO 99/24413). Nachteilig an diesem Verfahren ist, dass das als Ausgangsmaterial benötigte 2-Cyano-anilin nur durch aufwendige Synthesen zugänglich ist: So ist es beispielsweise erforderlich, zunächst Anthranilsäureamid in Gegenwart von Dimethylformamid mit Phosgen umzusetzen und das dabei entstehende N-2-Cyano-phenyl-N',N'-dimethyl-formamidinium-Hydrochlorid dann in einem zweiten Schritt mit Natriumacetat in wässrigem Medium zu behandeln (vgl. DE-A 2 115 624 und DE-A 2 115 625).

Nachteilig an dem in der WO 99/24413 beschriebenen Herstellungsverfahren ist auch, dass das Produkt durch aufwendige Aufarbeitungsmethoden isoliert werden muss (vgl. Beispiel 1 in der WO 99/24413).

Weiterhin ist bekannt, dass man 3,4-Dichlor-N-(2-cyano-phenyl)-5-isothiazolcarboxamid erhält, indem man
a) 3,4-Dichlorisothiazol-5-carbonsäurechlorid der Formel (II) 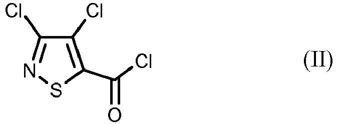 mit Anthranilsäureamid der Formel (III) 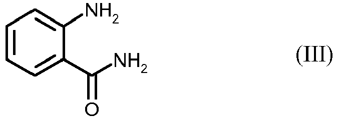 in Gegenwart eines Säureakzeptors und in Gegenwart eines aprotischen Verdünnungsmittels umsetzt und
b) dann das entstandene N-[2-(Aminocarbonyl)-phenyl]-3,4-dichlor-5-isothiazolcarboxamid der Formel (IV) 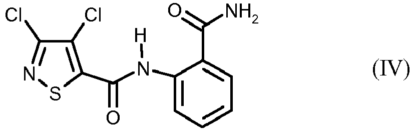 mit einem Dehydratisierungsmittel gegebenenfalls in Gegenwart eines zusätzlichen aprotischen Verdünnungsmittels umsetzt (vgl. WO 2004/002968).

Als geeignete Verdünnungsmittel bei der Durchführung beider Stufen des aus der WO 2004/002968 bekannten Verfahrens werden vorzugsweise gegebenenfalls halogenierte aromatische Kohlenwasserstoffe wie Toluol oder Chlorbenzol, ferner chlorierte Kohlenwasserstoffe wie Dichlormethan, Chloroform, Tetrachlorkohlenstoff, Dichlorethan oder Trichlorethan, und weiterhin Amide wie Dimethylformamid und Dimethylacetamid genannt. Als besonders bevorzugt wird Dimethylformamid angegeben.

Durch die Verwendung der genannten Verdünnungsmittel resultieren allerdings auch verschiedene Nachteile. So sind chlorierte Kohlenwasserstoffe wie Dichlormethan, Chloroform, Tetrachlorkohlenstoff, Dichlorethan oder Trichlorethan wegen ihres toxischen Potentials nur mit erhöhtem technischen Aufwand handhabbare Lösungsmittel. Wird die Reaktion in gegebenenfalls halogenierten aromatischen Kohlenwasserstoffen wie Toluol oder Chlorbenzol durchgeführt, wird vermehrt die Bildung von N-[2-(N'-Formyl-aminocarbonyl)-phenyl]-3,4-dichlor-5-isothiazolcarboxamid der Formel (V) 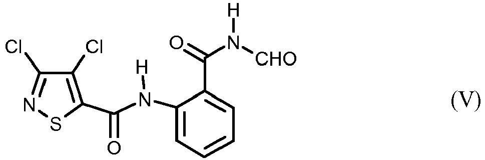 als unerwünschte Nebenkomponente beobachtet, durch welches das Produkt verunreinigt wird. Außerdem erfordert die Reaktion in diesen Verdünnungsmitteln erhöhte Mengen an Dehydratisierungsmittel.

Bei der Verwendung von Dimethylformamid als Verdünnungsmittel entsteht diese Nebenkomponente in deutlich geringerer Menge, kann aber auch hier nicht völlig verhindert werden. Außerdem ist die Verwendung von Dialkylamiden wie Dimethylformamid oder Dibutylformamid wegen des relativ hohen Preises dieser Lösungsmittel nachteilig. Dieser macht für ein ökonomisches Verfahren eine zumindest teilweise Rückgewinnung der Dialkylamid-Lösungsmittel erforderlich, was infolge der Eigenschaften der Dialkylamid-Lösungsmittel (wie völlige oder teilweise Mischbarkeit mit Wasser; hoher Siedepunkt) schwierig ist und einen erheblichen kostenintensiven zusätzlichen technischen Aufwand erfordert.

Es bestand daher Bedarf an einem verbesserten Verfahren, durch das 3,4-Dichlor-N-(2-cyano-phenyl)-5-isothiazolcarboxamid in guten Ausbeuten mit wesentlich verringerten Anteilen der Verunreinigung N-[2-(N'-Formyl-aminocarbonyl)-phenyl]-3,4-dichlor-5-isothiazolcarboxamid der Formel (V) ohne Verwendung eines großen Überschusses an Dialkylamid als Verdünnungsmittel hergestellt werden kann.

Demgemäß betrifft die vorliegende Erfindung ein Verfahren zur Herstellung von 3,4-Dichlor-N-(2-cyano-phenyl)-5-isothiazolcarboxamid der Formel (I) 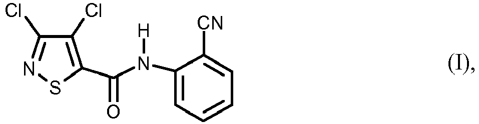 indem man
a) 3,4-Dichlorisothiazol-5-carbonsäurechlorid der Formel (II) 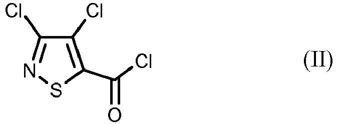 mit Anthranilsäureamid der Formel (III) 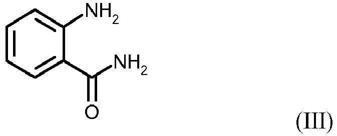 gegebenenfalls in Gegenwart eines Säureakzeptors umsetzt und
b) dann das entstandene N-[2-(Aminocarbonyl)-phenyl]-3,4-dichlor-5-isothiazolcarboxamid der Formel (IV) 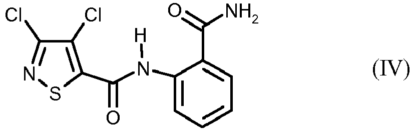 mit einem Dehydratisierungsmittel umsetzt,
   wobei das in Verfahrensschritt b) erhaltene Produkt in Gegenwart eines Alkohols aufgearbeitet und isoliert wird.

Ohne hiermit eine end- oder alleingültige Erklärung der Entstehungsweise von N-[2-(N'-Formyl-aminocarbonyl)-phenyl]-3,4-dichlor-5-isothiazolcarboxamid der Formel (V) geben zu wollen, scheint es jedoch möglich zu sein, dass diese Nebenkomponente erst bei der wässrigen Aufarbeitung des Reaktionsgemisches aus einer entsprechenden Vorstufe entsteht (vgl. dazu auch: J. Liebscher und A. Rumler, J. prakt. Chem. 326 (1984) 311-319). Es hat sich daher als vorteilhaft herausgestellt, das rohe 3,4-Dichlor-N-(2-cyano-phenyl)-5-isothiazolcarboxamid vor der wässrigen Aufarbeitung mit einem Alkohol zu vermischen und so die Vorstufe zum N-[2-(N'-Formyl-aminocarbonyl)-phenyl]-3,4-dichlor-5-isothiazolcarboxamid in ein alkohollöslisches Produkt zu überführen.

Als Alkohole für diese Ausführungsform des erfindungsgemäßen Verfahrens kommen grundsätzlich einwertige Alkohole sowie auch Diole in Frage. Aus Gründen der Verfügbarkeit und des Preises wird man bevorzugt einwertige C₁- bis C₆-Alkohole verwenden. Besonders bevorzugt sind Methanol und Ethanol.

Die Durchführung dieser Aufarbeitungsmethode mit einem Alkohol kann auf verschiedene Weise erfolgen. So ist es beispielsweise möglich, das Reaktionsgemisch zu filtrieren und den erhaltenen Feststoff zuerst mit einem Alkohol und dann mit Wasser zu waschen. Es ist jedoch auch möglich, den Alkohol nach erfolgter Umsetzung in das Reaktionsgemisch zu geben, anschließend mit Wasser zu versetzen und dann abzusaugen.

Da grundsätzlich unter den sauren Bedingungen des Reaktionsgemisches auch unerwünschte Nebenreaktionen des Produktes mit einem Alkohol möglich sind, so z.B. eine Pinner-Reaktion (siehe beispielsweise: R. Roger und D. G. Neilson, Chem. Rev. 61 (1961) 179-211), erfolgt die Durchführung der Aufarbeitungsmethode nach dem erfindungsgemäßen Verfahren gemäß zweiter Ausführungsform bei -10 bis 30°C; bevorzugt bei 0 bis 20°C.

Aus dem gleichen Grund erfolgt die Durchführung der Aufarbeitungsmethode nach dem erfindungsgemäßen Verfahren innerhalb einer kurzen Zeitspanne. Üblicherweise bringt man das Reaktionsgemisch für 10 Minuten bis 4 Stunden mit dem Alkohol in Kontakt; bevorzugt ist eine Zeit von 30 Minuten bis 2 Stunden.

Erfindungsgemäß wurde zusätzlich gefunden, dass man 3,4-Dichlor-N-(2-cyano-phenyl)-5-isothia-zolcarboxamid in sehr guten Ausbeuten und deutlich verbesserter Reinheit erhält, wenn man in dem Verfahren Essigsäuremethylester, Essigsäureethylester oder Mischungen davon als Verdünnungsmittel verwendet und bei der Aufarbeitung und Isolierung des Produkts einen Alkohol verwendet.

Demgemäß betrifft die vorliegende Erfindung in einer zweiten Ausführungsform ein Verfahren zur Herstellung von 3,4-Dichlor-N-(2-cyano-phenyl)-5-isothiazolcarboxamid der Formel (I) 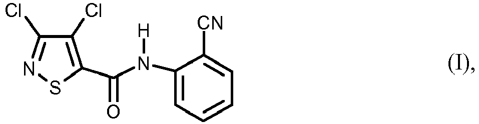 indem man
a) 3,4-Dichlorisothiazol-5-carbonsäurechlorid der Formel (II) 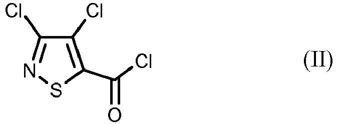 mit Anthranilsäureamid der Formel (III) 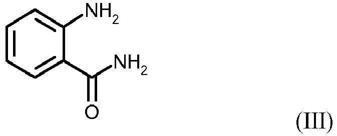 gegebenenfalls in Gegenwart eines Säureakzeptors umsetzt und
b) dann das entstandene N-[2-(Aminocarbonyl)-phenyl]-3,4-dichlor-5-isothiazolcarboxamid der Formel (IV) 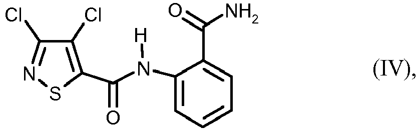 wobei zumindest der Verfahrensschritt b) in Gegenwart von Essigsäuremethylester, Essigsäureethylester oder einer Mischung davon als Verdünnungsmittel durchgeführt wird und das in Verfahrensschritt b) erhaltene Produkt in Gegenwart eines Alkohols aufgearbeitet und isoliert wird.

Das erfindungsgemäße Verfahren (im folgenden jeweils auf beide Ausführungsformen bezogen) kann als Eintopf-Reaktion durchgeführt werden, d.h. die Verfahrensschritte a) und b) werden unmittelbar hintereinander ohne Isolierung des Zwischenprodukts (IV) durchgeführt. Bei dieser Verfahrensweise, die besonders bevorzugt ist, wird das erfindungsgemäß vorgesehene Verdünnungsmittel aus Essigsäuremethylester. Essigsäureethylester oder einer Mischung davon auch schon bei der Durchführung von Verfahrensschritt a) verwendet.

Das erfindungsgemäße Verfahren kann auch als zweistufiges Verfahren durchgeführt werden, d.h. dass das Zwischenprodukt (IV) nach dem Verfahrensschritt a) zunächst isoliert und dann der Verfahrensschritt b) durchgeführt wird. Bei dieser Verfahrensweise wird zumindest der Verfahrensschritt b) in Gegenwart der erfingdungsgemäß vorgesehenen Verdünnungsmittel aus Essigsäuremethylester, Essigsäureethylester oder einer Mischung davon durchgeführt. Alternativ ist bei dieser zweistufigen Verfahrensweise auch die Verwendung der erfingdungsgemäß vorgesehenen Verdünnungsmittel in beiden Verfahrensschritten a) und b) möglich.

Besonders bevorzugt ist es, wenn als Verdünnungsmittel Essigsäuremethylester verwendet wird.

Der Verfahrensschritt (a) des erfindungsgemäßen Verfahrens wird gegebenenfalls in Gegenwart eines Säureakzeptors durchgeführt.

Wenn ein Säureakzeptors in Verfahrensschritt a) verwendet wird, kommen vorzugsweise tertiäre Amine in Frage. Beispielhaft benannt seien Trimethylamin_{.} Triethylamin, Tributylamin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Wenn ein Säureakzeptor bei der Durchführung des erfindungsgemäßen Verfahrens verwendet wird, beiträgt die Mengen in Säureakzeptor im allgemeinen 0,5 bis 1,5 Mol pro Mol an 3,4-Dicllor-isothiazol-5-carbonsäurechlorid. Darüber hinaus setzt man bei der Durchführung der ersten Stufe des erfindungsgemäßen Verfahrens auf 1 Mol an 3,4-Dichlor-isothiazol-5-carbotisäurechlorid der Formel (II im allgemeinen zwischen 1 und 1,5 Mol an Anthranilsäureamid der Formel (III) ein.

Die Temperaturen können bei der Durchführung des erfindungsgemäßen Verfahrens innerhalb eines größere Bereiches variieren. Die Temperaturen bei der Durchführung des erfindungsgemäßen Verfahrens siegen im allgemeinen im Bereich zwischen -20 und 160 °C. Vorzugsweise arbeitet man zwischen -10 und 40 °C, besonders bevorzugt zwischen 0 und 25 °C, ganz besonders bevorzugt bei Raumtemperatur.

Es ist bevorzugt, wenn beide Verfahrensstufen a) und b) des erfindungsgemäßen Verfahrens bei gleicher Temperatur durchgeführt werden. Alternativ ist es aber auch möglich, die beiden Verfahrenschritte a) und b) des erfindungsgemäßen Verfahrens bei unterschiedlichen Temperaturen durchzuführen. Dabei kann Verfahrensschritt a) bei einer Temperatur von -20 bis 160 °C durchgeführt werden. Verfahrensschritt b) wird dann vorzugsweise bei einer Temperatur von -10 bis 30 °C durchgeführt.

Verfahrensschritt (b) wird in Gegenwart eines Dehydratisierungsmittels durchgeführt.

Als Dehydratisierungsmittel kommen vorzugsweise Reagenzien in Frage, die ausgewählt sind aus der Gruppe, bestehend aus Mischungen von Dialkylformamid, insbesondere Dimethylformamid und Dibutylformamid, mit Thionylchlorid. Phosphoroxychlorid, Phosgen und/oder Chlormethylen-dimethylammoniumchlorid. Ganz besonders bevorzugt ist die Verwendung eines Gemisches aus Dimethylformamid oder Dibutylformamid und Thionylchlorid oder Phosgen als Dehydratisierungsmittel.

Die bei der Durchführung des erfindungsgemäßen Verfahrens eingesetzten Mengen an Phosgen oder Thionylchlorid liegen im allgemeinen zwischen 1 und 6 Mol je 1 Mol 3,4-Dichlor-isothiazol-5-carbonsäurechlorid. Bevorzugt verwendet man zwischen 2 und 4 Mol je 1 Mol 3,4-Diclvlor-isothiazol-5-carbonsäurechlorid.

Die bei der Durchführung des erfindungsgemäßen Verfahrens eingesetzten Mengen an Dialkylformamid liegen zwischen 1 und 4 Mol je 1 Mol 3,4-Dichlor-isothiazol-5-carbonsäurechlorid. Bevorzugt verwendet man zwischen 2 und 3 Mol je 1 Mol 3,4-Dichlor-isothiazol-5-carbonsäurechlorid.

Darüber hinaus kann in dem erfindungsgemäßen Verfahren die Umsetzung gemäß erster und/oder zweiter Verfahrensstufe in Gegenwart eines weiteren Verdünnungsmittels durchgeführt werden. Als weitere Verdünnungsmittel können bei der Durchführung des erfindungsgemäßen Verfahrens alle üblichen aprotischen, organischen Solventien eingesetzt werden. Vorzugsweise verwendbar sind gegebenenfalls halogenierte aromatische Kohlenwasserstoffe wie Toluol oder Chlorbenzol, ferner chlorierte Kohlenwasserstoffe wie Dichlormethan, Chloroform, Tetrachlorkohlenstoff. Dichlorethan oder Trichlorethan und weiterhin Amide wie Dialkylformamid und Dialkylacetamid. Als Dialkylformamid können beispielsweise Dimethylformamid. Diethylformamid, Dipropylformamid, Dibutylformamid oder N-Formyl-morpholin ein gesetzt werden. Bevorzugt sind Dimethylformamid und Dibutylformamid, besonders bevorzugt Dimethylformamid. Als Dialkylacetamid wird vorzugsweise Dimethylacetamid verwendet.

Bei der Durchführung des erfindungsgemäßen Verfahrens arbeitet man sowohl bei der Umsetzung in der ersten Stufe als auch in der zweiten Stufe im allgemeinen unter Atmosphärendruck. Es ist jedoch auch möglich, unter erhöhtem Druck zu arbeiten.

Die Reaktionszeiten bei der Durchführung des erfindungsgemäßen Verfahrens liegen im allegemeinen zwischen 1 und 24 Stunden und sind im Wesentlichen von der Reaktionstemperatur sowie der Wahl und der Menge an jeweils verwendetem Säureakzeptor und Dehydratisierungsmittel abhängig. Bevorzugte Reaktionszeiten liegen zwischen 2 und 8 Stunden.

Das erfindungsgemäße Verfahren zeichnet sich durch eine Reihe von Vorteilen aus. Es ermöglicht die Herstellung von 3,4-Dichlor-N-(2-cyano-phenyl)-5-isothiazolcarboxamid in sehr guter Ausbeute und sehr hoher Reinheit. Das erfindungsgemäße Verfahren lässt sich problemlos in den technischen Maßstab übertragen.

Die vorliegende Erfindung wird anhand der nachfolgend beschriebenen Beispiele näher erläutert, welche jedoch die vorliegende Erfindung nicht beschränken. Die Ausbeuteangaben verstehen sich dabei als Gesamtausbeute über beide Verfahrensstufen.

### Ausführungs- und Vergleichsbeispiele

### Beispiel 1

In 1280 ml Essigsäuremethylester werden 119,8 g [0,88 mol] Anthranilsäureamid, 89,1 g [0,88 mol] Triethylamin und 175,4 g [2,4 mol] N,N-Dimethylformamid (DMF) vorgelegt. Bei 10-20°C wird eine Lösung von 163,7 g [0,756 mol] 3,4-Dichlorisothiazol-5-carbonsäurechlorid in 320 ml Essigsäuremethylester zugetropft. Man rührt 1 Stunde bei 10-20°C und tropft dann bei gleicher Temperatur 380,7 g [3,2 mol] Thionylchlorid innerhalb von 30 Minuten zu. Nach 2 Stunden bei 20°C wird das Reaktionsgemisch filtriert und der Feststoff auf der Nutsche nacheinander mit 200 ml Essigsäuremethylester, zweimal je 200 ml Methanol, dreimal je 400 ml Wasser und nochmals 200 ml Methanol gewaschen. Nach Trocknung erhält man 198,8 g beigen Feststoff folgender Zusammensetzung:
97,4% 3,4-Dichlor-N-(2-cyano-phenyl)-5-isothiazolcarboxamid (85,9% d.Th.)
0,43% N-[2-(Aminocarbonyl)-phenyl]-3,4-dichlor-5-isothiazolcarboxamid
0,42% N-[2-(N'-Formyl-aminocarbonyl)-phenyl]-3,4-dichlor-5-isothiazol-carboxamid

### Beispiel 2

In 80 ml Essigsäuremethylester werden 7,49 g [55 mmol] Anthranilsäureamid, 5,57 g [55 mmol] Triethylamin und 11 g [150 mmol] DMF vorgelegt. Bei 10-20°C wird eine Lösung von 10,2 g [47,2 mmol] 3,4-Dichlorisothiazol-5-carbonsäurechlorid in 20 ml Essigsäuremethylester zugetropft. Man rührt 1 Stunde bei 10-20°C und tropft danach bei gleicher Temperatur 23,8 g [200 mmol] Thionylchlorid innerhalb von 15 Minuten zu. Nach 2 Stunden bei 20°C gibt man unter Kühlung 50 ml Methanol zum Reaktionsgemisch, rührt 30 Minuten, saugt den Feststoff ab und wäscht ihn auf der Nutsche nacheinander mit zweimal je 20 ml Wasser und einmal 20 ml Methanol. Nach Trocknung erhält man 12,34 g beigen Feststoff folgender Zusammensetzung:
97,5% 3,4-Dichlor-N-(2-cyano-phenyl)-5-isothiazolcarboxamid (85,5% d.Th.)
0,46% N-[2-(Aminocarbonyl)-phenyl]-3,4-dichlor-5-isothiazolcarboxamid
0,37% N-[2-(N'-Formyl-aminocarbonyl)-phenyl]-3,4-dichlor-5-isothiazol-carboxamid

### Beispiel 3

In 320 ml Essigsäuremethylester werden 29,95 g [220 mmol] Anthranilsäureamid, 22,26 g [220 mmol] Triethylamin und 43,86 g [600 mmol] DMF vorgelegt. Bei 10-20°C wird eine Lösung von 43,3 g [200 mmol] 3,4-Dichlorisothiazol-5-carbonsäurechlorid in 80 ml Essigsäuremethylester zugetropft. Man rührt 1 Stunde bei 10-20°C und tropft dann bei gleicher Temperatur 95,2g [800 mmol] Thionylchlorid innerhalb von 2 Stunden zu. Anschließend rührt man noch 30 Minuten bei 20°C und gibt dann unter Kühlung 200 ml Methanol zum Reaktionsgemisch. Nach 30 Minuten Rühren versetzt man unter Kühlung mit 200 ml Wasser und verrührt das Gemisch für weitere 2 Stunden. Dann saugt man den Feststoff ab und wäscht ihn auf der Nutsche nacheinander mit 50 ml Wasser und zweimal 50 ml Methanol. Nach Trocknung erhält man 54,8 g beigen Feststoff folgender Zusammensetzung:
96,75% 3,4-Dichlor-N-(2-cyano-phenyl)-5-isothiazolcarboxamid (88,9% d.Th.)
0,45% N-[2-(Aminocarbonyl)-phenyl]-3,4-dichlor-5-isothiazolcarboxamid
0,44% N-[2-(N'-Formyl-aminocarbonyl)-phenyl]-3,4-dichlor-5-isothiazol-carboxamid

### Beispiel 4

In 320 ml Essigsäuremethylester werden 29,95 g [220 mmol] Anthranilsäureamid, 22,26 g [220 mmol] Triethylamin und 43,86 g [600 mmol] DMF vorgelegt. Bei 10-20°C wird eine Lösung von 43,3 g [200 mmol] 3,4-Dichlorisothiazol-5-carbonsäurechlorid in 80 ml Essigsäuremethylester zugetropft. Man rührt 1 Stunde bei 10-20°C und tropft anschließend bei gleicher Temperatur 95,2 g [800 mmol] Thionylchlorid innerhalb von 2 Stunden zu. Danach rührt man noch für 2 Stunden bei 20°C und gibt dann unter Kühlung 200 ml Methanol zum Reaktionsgemisch. Nach 30 Minuten Rühren versetzt man unter Kühlung mit 200 ml Wasser und verrührt das Gemisch für weitere 2 Stunden. Man saugt den Feststoff ab und wäscht ihn auf der Nutsche nacheinander mit 50 ml Wasser und zweimal 50 ml Methanol. Nach Trocknung resultieren 54,4 g beiger Feststoff folgender Zusammensetzung:
96,6% 3,4-Dichlor-N-(2-cyano-phenyl)-5-isothiazolcarboxamid (88,1% d.Th.)
0,54% N-[2-(Aminocarbonyl)-phenyl]-3,4-dichlor-5-isothiazolcarboxamid
0,35% N-[2-(N'-Formyl-aminocarbonyl)-phenyl]-3,4-dichlor-5-isothiazol-carboxamid

### Beispiel 5

In 80 ml Essigsäuremethylester werden 7,49 g [55 mmol] Anthranilsäureamid, 5,57 g [55 mmol] Triethylamin und 11 g [150 mmol] DMF vorgelegt. Bei 10-20°C wird eine Lösung von 10,83 g [50 mmol] 3,4-Dichlorisothiazol-5-carbonsäurechlorid in 20 ml Essigsäuremethylester zugetropft. Man rührt 1 Stunde bei 10-20°C und tropft dann bei gleicher Temperatur 23,8 g [200 mmol] Thionylchlorid innerhalb von 15 Minuten zu. Nach 4 Stunden bei 20°C versetzt man das Reaktionsgemisch unter Kühlung bei 0-5°C mit 100 ml Wasser, verrührt für 15 Minuten, saugt den Feststoff ab und wäscht ihn auf der Nutsche zweimal mit je 20 ml Isopropanol. Nach Trocknung erhält man 13,39 g beigen Feststoff mit folgender Zusammensetzung:
98,1% 3,4-Dichlor-N-(2-cyano-phenyl)-5-isothiazolcarboxamid (88,1% d.Th.)
0,2% N-[2-(Aminocarbonyl)-phenyl]-3,4-dichlor-5-isothiazolcarboxamid
0,4% N-[2-(N'-Formyl-aminocarbonyl)-phenyl]-3,4-dichlor-5-isothiazolcarboxamid

### Beispiel 6

In 80 ml Essigsäuremethylester werden 7,49 g [55 mmol] Anthranilsäureamid, 5,57 g [55 mmol] Triethylamin und 7,31 g [100 mmol] DMF vorgelegt. Bei 10-20°C wird eine Lösung von 10,83 g [50 mmol] 3,4-Dichlorisothiazol-5-carbonsäurechlorid in 20 ml Essigsäuremethylester zugetropft. Man rührt 1 Stunde bei 10-20°C und tropft danach bei gleicher Temperatur 11,9 g [100 mmol] Thionylchlorid innerhalb von 15 Minuten zu. Nach 4 Stunden bei 20°C versetzt man das Reaktionsgemisch unter Kühlung mit 100 ml Wasser, verrührt für 15 Minuten, saugt den Feststoff ab und wäscht ihn auf der Nutsche mit 50 ml Wasser und zweimal je 20 ml Isopropanol. Nach Trocknung erhält man 13,35 g beigen Feststoff mit folgender Zusammensetzung:
98,1% 3,4-Dichlor-N-(2-cyano-phenyl)-5-isothiazolcarboxamid (87,9% d.Th.)
0,4% N-[2-(Aminocarbonyl)-phenyl]-3,4-dichlor-5-isothiazolcarboxamid
< 0,05% N-[2-(N'-Formyl-aminocarbonyl)-phenyl]-3,4-dichlor-5-isothiazol-carboxamid

### Beispiel 7

In 8 ml Essigsäure-ethylester werden 0,749 g [5,5 mmol] Anthranilsäureamid, 0,557 g [5,5 mmol] Triethylamin und 1,1 g [15 mmol] DMF vorgelegt. Bei 0°C wird eine Lösung von 1,08 g [5 mmol] 3,4-Dichlorisothiazol-5-carbonsäurechlorid in 2 ml Essigsäureethylester zugetropft. Man rührt 1 Stunde bei 0°C und tropft danach bei gleicher Temperatur 2,38 g [20 mmol] Thionylchlorid innerhalb von 15 Minuten zu. Nach 2 Stunden bei 0°C und 2 Stunden bei 20°C versetzt man das Reaktionsgemisch unter Kühlung bei 0°C mit 10 ml Wasser, verrührt für 15 Minuten, saugt den Feststoff ab und wäscht ihn auf der Nutsche mit 20 ml Wasser. Nach Trocknung erhält man 1,34 g beigen Feststoff mit folgender Zusammensetzung:
97,2% 3,4-Dichlor-N-(2-cyano-phenyl)-5-isothiazolcarboxamid (87,2% d.Th.)
0,2% N-[2-(Aminocarbonyl)-phenyl]-3,4-dichlor-5-isothiazolcarboxamid
0,4% N-[2-(N'-Formyl-aminocarbonyl)-phenyl]-3,4-dichlor-5-isothiazol-carboxamid

### Beispiel 8

Es wird vorgegangen wie in Beispiel 4 beschrieben, mit dem Unterschied, dass Essigsäure-ethylester als Verdünnungsmittel verwendet wird. Man erhält 50,9 g beigen Feststoff folgender Zusammensetzung:
97,0% 3,4-Dichlor-N-(2-cyano-phenyl)-5-isothiazolcarboxamid (82,8% d.Th.)
0,30% N-[2-(Aminocarbonyl)-phenyl]-3,4-dichlor-5-isothiazolcarboxamid
0,51% N-[2-(N'-Formyl-aminocarbonyl)-phenyl]-3,4-dichlor-5-isothiazol-carboxamid

### Beispiel 9

In 80 ml Essigsäuremethylester werden 7,49 g [55 mmol] Anthranilsäureamid, 5,57 g [55 mmol] Triethylamin und 11 g [150 mmol] DMF vorgelegt. Bei 10-20°C wird eine Lösung von 10,22 g [47,2 mmol] 3,4-Dichlorisothiazol-5-carbonsäurechlorid in 20 ml Essigsäure-ethylester zugetropft. Man rührt 1 Stunde bei 20°C und tropft danach bei 10-20°C 23,8 g [200 mmol] Thionylchlorid innerhalb von 15 Minuten zu. Es wird 16 Stunden bei 20°C gerührt. Danach wird der Feststoff durch Filtration isoliert und in 2 Hälften geteilt. Die 1. Hälfte des Feststoffes wird auf dem Filter mit zweimal je 10 ml Ethanol, dann dreimal je 15 ml Wasser und noch einmal 10 ml Ethanol gewaschen. Nach Trocknen erhält man 4,8 beigen Feststoff folgender Zusammensetzung:
97,2% 3,4-Dichlor-N-(2-cyano-phenyl)-5-isothiazolcarboxamid
0,34% N-[2-(Aminocarbonyl)-phenyl]-3,4-dichlor-5-isothiazolcarboxamid
0,31% N-[2-(N'-Formyl-aminocarbonyl)-phenyl]-3,4-dichlor-5-isothiazol-carboxamid

Die 2. Hälfte des Feststoffes wird entsprechend mit n-Butanol behandelt. Es resultieren 4 g Feststoff folgender Zusammensetzung:
97,4% 3,4-Dichlor-N-(2-cyano-phenyl)-5-isothiazolcarboxamid
0,35% N-[2-(Aminocarbonyl)-phenyl]-3,4-dichlor-5-isothiazolcarboxamid
0,36% N-[2-(N'-Formyl-aminocarbonyl)-phenyl]-3,4-dichlor-5-isothiazol-carboxamid

### Vergleichsbeispiel 1

Man geht vor wie in Beispiel 5 beschrieben, mit dem Unterschied, dass anstelle von Essigsäuremethylester Essigsäureisopropylester verwendet wird. Man erhält 15 g beigen Feststoff mit folgender Zusammensetzung:
81,5% 3,4-Dichlor-N-(2-cyano-phenyl)-5-isothiazolcarboxamid (82% d.Th.)
0,3% N-[2-(Aminocarbonyl)-phenyl]-3,4-dichlor-5-isothiazolcarboxamid
13,2% N-[2-(N'-Formyl-aminocarbonyl)-phenyl]-3,4-dichlor-5-isothiazol-carboxamid

### Vergleichsbeispiel 2

Es wird vorgegangen wie in Beispiel 5 beschrieben, mit dem Unterschied, dass die Reaktion in Propionsäureethylester durchgeführt wird.

Man erhält 14,5 g beigen Feststoff folgender Zusammensetzung:
83,5% 3,4-Dichlor-N-(2-cyano-phenyl)-5-isothiazolcarboxamid (81,2% d.Th.)
0,3% N-[2-(Aminocarbonyl)-phenyl]-3,4-dichlor-5-isothiazolcarboxamid
13,4% N-[2-(N'-Formyl-aminocarbonyl)-phenyl]-3,4-dichlor-5-isothiazol-carboxamid

### Vergleichsbeispiel 3

Es wird vorgegangen wie in Beispiel 4 beschrieben, mit dem Unterschied, dass die Reaktion in Ameisensäuremethylester durchgeführt wird.

Man erhält 7,6 g beigen Feststoff mit nach NMR folgender Zusammensetzung:
98% 3,4-Dichlor-N-(2-cyano-phenyl)-5-isothiazolcarboxamid (50% d.Th.)
2% N-[2-(Aminocarbonyl)-phenyl]-3,4-dichlor-5-isothiazolcarboxamid

### Vergleichsbeispiel 4

In 30 g Dimethylformamid (DMF) werden 7,49 g [50 mmol] Anthranilsäureamid und 5,57 g [55 mmol] Triethylamin vorgelegt. Bei 0-5°C wird eine Lösung von 10,83 g [50 mmol] 3,4-Dichloriso-thiazol-5-carbonsäurechlorid in 25 g DMF zugetropft. Man rührt 1 Stunde bei 0-5°C und tropft dann bei gleicher Temperatur 8,63 g [72,5 mmol] Thionylchlorid innerhalb von 15 Minuten zu. Nach 4 Stunden bei 0-5°C wird das Reaktionsgemisch mit 100 ml Wasser versetzt und weitere 4 Stunden gerührt. Dann wird der Feststoff abgesaugt, einmal mit 20 ml Wasser und zweimal mit je 20 ml Isopropanol gewaschen und getrocknet.

Man erhält 14,05 g beigen Feststoff folgender Zusammensetzung:
96,2% 3,4-Dichlor-N-(2-cyano-phenyl)-5-isothiazolcarboxamid (90,7% d.Th.)
0,5% N-[2-(Aminocarbonyl)-phenyl]-3,4-dichlor-5-isothiazolcarboxamid
2,1% N-[2-(N'-Formyl-aminocarbonyl)-phenyl]-3,4-dichlor-5-isothiazolcarboxamid

### Vergleichsbeispiel 5

In 80 ml Chlorbenzol werden 7,49 g [50 mmol] Anthranilsäureamid, 5,57 g [55 mmol] Triethylamin und 14,62 g [200 mmol] DMF vorgelegt. Bei 20°C wird eine Lösung von 10,83 g [50 mmol] 3,4-Dichlorisothiazol-5-carbonsäurechlorid in 20 ml Chlorbenzol zugetropft. Man rührt 1 Stunde bei 20°C, kühlt das Reaktionsgemisch dann auf 0°C ab und tropft bei gleicher Temperatur 23,79 g [200 mmol] Thionylchlorid innerhalb von 20 Minuten zu. Nach 6 Stunden bei 0-5°C wird das Reaktionsgemisch mit 100 ml Wasser versetzt und 15 Minuten verrührt. Der Feststoff wird abgesaugt, zweimal mit je 20 ml Isopropanol gewaschen und getrocknet.

Man erhält 14,45 g beigen Feststoff mit n. NMR folgender Zusammensetzung:
48% 3,4-Dichlor-N-(2-cyano-phenyl)-5-isothiazolcarboxamid (46% d.Th.)
52% N-[2-(Aminocarbonyl)-phenyl]-3,4-dichlor-5-isothiazolcarboxamid
<0,5% N-[2-(N'-Formyl-aminocarbonyl)-phenyl]-3,4-dichlor-5-isothiazol-carboxamid

### Vergleichsbeispiel 6

In 80 ml Chlorbenzol werden 7,49 g [50 mmol] Anthranilsäureamid, 5,57 g [55 mmol] Triethylamin und 14,62 g [200 mmol] DMF vorgelegt. Bei 20°C wird eine Lösung von 10,83 g [50 mmol] 3,4-Dichlorisothiazol-5-carbonsäurechlorid in 20 ml Chlorbenzol zugetropft. Man rührt 1 Stunde bei 20°C, kühlt das Reaktionsgemisch dann auf 10°C ab und tropft bei gleicher Temperatur 23,79 g [200 mmol] Thionylchlorid innerhalb von 20 Minuten zu. Nach 6 Stunden bei 10°C wird das Reaktionsgemisch mit 100 ml Wasser versetzt und 15 Minuten verrührt. Der Feststoff wird abgesaugt, zweimal mit je 20 ml Isopropanol gewaschen und getrocknet.

Man erhält 13,56 g beigen Feststoff mit n. NMR folgender Zusammensetzung:
92% 3,4-Dichlor-N-(2-cyano-phenyl)-5-isothiazolcarboxamid (84% d.Th.)
2% N-[2-(Aminocarbonyl)-phenyl]-3,4-dichlor-5-isothiazolcarboxamid
6% N-[2-(N'-Formyl-aminocarbonyl)-phenyl]-3,4-dichlor-5-isothiazolcarboxamid

### Vergleichsbeispiel 7

In 40 ml Butyronitril werden 7,49 g [50 mmol] Anthranilsäureamid, 5,57 g [55 mmol] Triethylamin und 11 g [150 mmol] DMF vorgelegt. Bei 0-5°C wird eine Lösung von 10,83 g [50 mmol] 3,4-Dichlorisothiazol-5-carbonsäurechlorid in 10 ml Butyronitril zugetropft. Man rührt 1 Stunde bei 0-5°C und tropft bei gleicher Temperatur 23,79 g [200 mmol] Thionylchlorid innerhalb von 20 Minuten zu. Nach 5 Stunden bei 0-5°C und 1 Stunde bei 20°C wird das Reaktionsgemisch mit 100 ml Wasser versetzt und 15 Minuten verrührt. Der Feststoff wird abgesaugt, zweimal mit je 20 ml Isopropanol gewaschen und getrocknet.

Man erhält 12,25 g beigen Feststoff mit n. NMR folgender Zusammensetzung:
96% 3,4-Dichlor-N-(2-cyano-phenyl)-5-isothiazolcarboxamid (79% d.Th.)
1% N-[2-(Aminocarbonyl)-phenyl]-3,4-dichlor-5-isothiazolcarboxamid
0,4% N-[2-(N'-Formyl-aminocarbonyl)-phenyl]-3,4-dichlor-5-isothiazolcarboxamid

## Patentansprüche

1. Verfahren zur Herstellung von 3,4-Dichlor-N-(2-cyano-phenyl)-5-isothiazolcarboxamid der Formel (I) 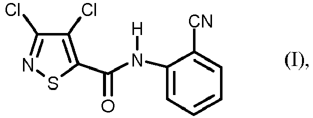 indem man
a) 3,4-Dichlorisothiazol-5-carbonsäurechlorid der Formel (II) 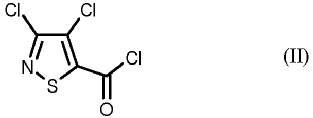 mit Anthranilsäureamid der Formel (III) 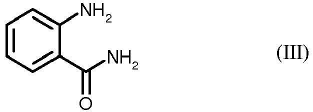 gegebenenfalls in Gegenwart eines Säureakzeptors umsetzt und
b) dann das entstandene N-[2-(Aminocarbonyl)-phenyl]-3,4-dichlor-5-isothiazolcarboxamid der Formel (IV) 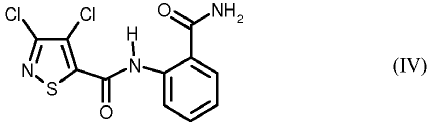 mit einem Dehydratisierungsmittel umsetzt,
**dadurch gekennzeichnet, dass** das in Verfahrensschritt b) erhaltene Produkt in Gegenwart eines Alkohols aufgearbeitet und isoliert wird.

2. Verfahren nach Anspruch 1, **dadurch gekennzeichnet, dass** zumindest der Verfahrensschritt b) in Gegenwart von Essigsäuremethylester, Essigsäureethylester oder einer Mischung davon als Verdünnungsmittel durchgeführt wird.

3. Verfahren nach Anspruch 1 oder 2, **dadurch gekennzeichnet, dass** das Verfahren ohne Isolierung des Zwischenprodukts (IV) durchgeführt wird und der als Verdünnungsmittel verwendete Essigsäuremethylester, Essigsäureethylester oder die Mischung davon in beiden Verfahrensstufen verwendet wird.

4. Verfahren nach einem der Ansprüche 1 bis 3, **dadurch gekennzeichnet, dass** man die Aufarbeitung und Isolierung in Gegenwart des Alkohols bei einer Temperatur zwischen -10 und 30°C und/oder in einer Zeitspanne von 10 Minuten bis 2 Stunden durchführt.

5. Verfahren nach einem der Ansprüche 1 bis 4, **dadurch gekennzeichnet, dass** man die Aufarbeitung und Isolierung mit einem C₁- bis C₆-Alkohol durchführt.

6. Verfahren nach einem der Ansprüche 1 bis 5, **dadurch gekennzeichnet, dass** man bei der Durchführung der zweiten Verfahrensstufe ein Reagenz als Dehydratisierungsmittel einsetzt, dass ausgewählt ist aus der Gruppe, bestehend aus Mischungen von Dialkylformamid mit Thionylchlorid, Phosphoroxychlorid, Phosgen und/oder Chlormethylen-dimethylammoniumchlorid.

7. Verfahren nach einem der Ansprüche 1 bis 6, **dadurch gekennzeichnet, dass** man die Umsetzung gemäß Verfahrensstufe a) und b) bei einer Temperatur von -20 bis 60 °C durchgeführt wird.

8. Verfahren nach einem der Ansprüche 1 bis 7, **dadurch gekennzeichnet, dass** die eingesetzten Mengen an Phosgen oder Thionylchlorid in Verfahrensschritt b) zwischen 1 und 6 Mol je Mol 3,4-Dichlor-isothiazol-5-carbonsäurechlorid liegen.

9. Verfahren nach einem der Ansprüche 1 bis 8, **dadurch gekennzeichnet, dass** die eingesetzten Mengen an Dialkylformamid in Verfahrensschritt b) zwischen 2 und 4 Mol je 1 Mol 3,4-Dichlor-isothiazol-5-carbonsäurechlorid liegen.

## Claims

1. Process for preparing 3,4-dichloro-N-(2-cyanophenyl)-5-isothiazolecarboxamide of the formula (I) 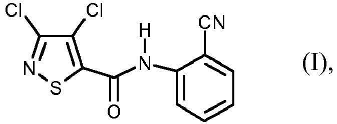 by
a) reacting 3,4-dichloroisothiazole-5-carbonyl chloride of the formula (II) 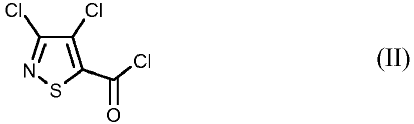 with anthranilamide of the formula (III) 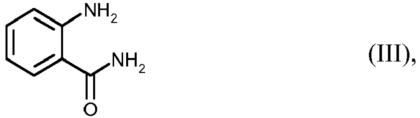 if appropriate in the presence of an acid acceptor, and
b) then reacting the N-[2-(aminocarbonyl)phenyl]-3,4-dichloro-5-isothiazolecarboxamide of the formula (IV) formed 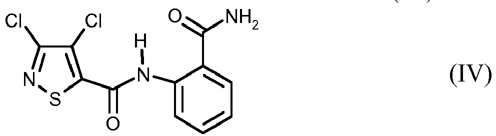 with a dehydrating agent,
**characterized in that** the product obtained in process step b) is worked up and isolated in the presence of an alcohol.

2. Process according to Claim 1, **characterized in that** at least process step b) is carried out in the presence of methyl acetate, ethyl acetate or a mixture thereof as diluent.

3. Process according to Claim 1 or 2, **characterized in that** the process is carried out without isolation of the intermediate (IV) and the methyl acetate, ethyl acetate or the mixture thereof used as diluent is used in both process steps.

4. Process according to any of Claims 1 to 3, **characterized in that** the work-up and isolation is carried out in the presence of an alcohol at a temperature between -10 and 30°C and/or over a period of from 10 minutes to 2 hours.

5. Process according to any of Claims 1 to 4, **characterized in that** the work-up and isolation is carried out using a C₁- to C₆-alcohol.

6. Process according to any of Claims 1 to 5, **characterized in that** the reagent used as dehydrating agent during the practice of the second process step is selected from the group consisting of mixtures of dialkylformamide with thionyl chloride, phosphorus oxychloride, phosgene and/or chloromethylenedimethylammonium chloride.

7. Process according to any of Claims 1 to 6, **characterized in that** the reaction according to process steps a) and b) is carried out at a temperature of from - 20 to 60°C.

8. Process according to any of Claims 1 to 7, **characterized in that** the amounts of phosgene or thionyl chloride employed in process step b) are between 1 and 6 mol per mole of 3,4-dichloroisothiazole-5-carbonyl chloride.

9. Process according to any of Claims 1 to 8, **characterized in that** the amounts of dialkylformamide employed in process step b) are between 2 and 4 mol per mole of 3,4-dichloroisothiazole-5-carbonyl chloride.

## Revendications

1. Procédé pour la préparation de 3,4-dichloro-N-(2-cyano-phényl)-5-isothiazole-carboxamide de formule (I) 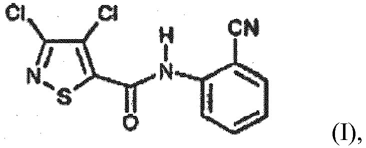 par
a) mise en réaction de chlorure de 3,4-dichloro-isothiazole-5-carbonyle de formule (II) 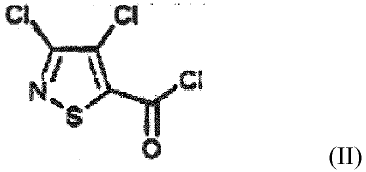 avec de l'anthranilamide de formule (III) 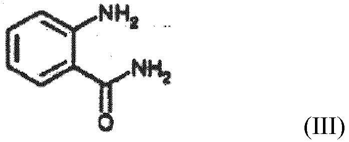 éventuellement en présence d'un accepteur d'acide et
b) ensuite mise en réaction du N-[2-(aminocarbonyl)-phényl]-3,4-dichloro-5-isothiazole-carboxamide résultant de formule (IV) 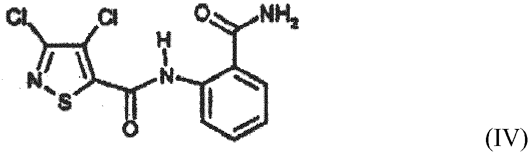 avec un agent de déshydratation,
**caractérisé en ce qu'**on traite et isole le produit obtenu dans l'étape b) due procédé en présence d'un alcool.

2. Procédé selon la revendication 1, **caractérisé en ce qu'**au moins l'étape b) du procédé est effectuée en présence d'acétate de méthyle, d'acétate d'éthyle ou d'un mélange de ceux-ci, en tant que diluant.

3. Procédé selon la revendication 1 ou 2, **caractérisé en ce que** le procédé est effectué sans isolement du produit intermédiaire (IV) et l'acétate de méthyle, l'acétate d'éthyle ou le mélange de ceux-ci, utilisé comme diluant, est utilisé dans les deux étapes du procédé.

4. Procédé selon l'une quelconque des revendications 1 à 3, **caractérisé en ce qu'**on effectue le traitement final et l'isolement en présence de l'alcool à une température entre -10 et 30 °C et/ou pendant une durée de temps de 10 minutes à 2 heures.

5. Procédé selon l'une quelconque des revendications 1 à 4, **caractérisé en ce qu'**on effectue le traitement final et l'isolement avec un alcool en C₁ à C₆.

6. Procédé selon l'une quelconque des revendications 1 à 5, **caractérisé en ce que** dans l'exécution de la deuxième étape du procédé on utilise comme agent de déshydratation un réactif qui est choisi dans le groupe constitué par des mélanges de dialkylformamide avec du chlorure de thionyle, de l'oxychlorure de phosphore, du phosgène et/ou du chlorure de chlorométhylène-diméthylammonium.

7. Procédé selon l'une quelconque des revendications 1 à 6, **caractérisé en ce qu'**on effectue la réaction selon les étapes a) et b) du procédé à une température de -20 à 60 °C.

8. Procédé selon l'une quelconque des revendications 1 à 7, **caractérisé en ce que** les quantités utilisées de phosgène ou de chlorure de thionyle dans l'étape b) du procédé sont comprises entre 1 et 6 moles par mole de 3,4-dichloro-isothiazo le-5 -carbonyle.

9. Procédé selon l'une quelconque des revendications 1 à 8, **caractérisé en ce que** les quantités utilisées de dialkylformamide dans l'étape b) du procédé sont comprises entre 2 et 4 moles par mole de 3,4-dichloro-isothiazole-5-carbonyle.
